# NEUE EUROPÄISCHE PATENTSCHRIFT

(11) **EP 1 797 061 B2**
(45) Veröffentlichungstag und Bekanntmachung des Hinweises auf die Entscheidung über den Einspruch: **26.10.2011**
(45) Hinweis auf die Patenterteilung: 19.03.2008
(21) Anmeldenummer: 05794724.4
(22) Anmeldetag: 21.09.2005
(51) Int. Cl.: C07D 307/08

(54) **VERFAHREN ZUR DESTILLATIVEN AUFARBEITUNG VON TETRAHYDROFURAN**
METHOD FOR THE DISTILLATIVE PROCESSING OF TETRAHYDROFURAN
PROCEDE DE TRAITEMENT PAR DISTILLATION DU TETRAHYDROFURANE

(30) Priorität: 29.09.2004 DE 102004047201
(43) Veröffentlichungstag der Anmeldung: 20.06.2007
(73) Patentinhaber: BASF SE, 67056 Ludwigshafen (DE)
(72) Erfinder: WINDECKER, Gunther, 67067 Ludwigshafen (DE); WECK, Alexander, 67251 Freinsheim (DE); RÖSCH, Markus, 55276 Dienheim (DE); STEINIGER, Michael, 67435 Neustadt (DE); PINKOS, Rolf, 67098 Bad Dürkheim (DE); VANDEN HAUTTE, Dany, B-7320 Bernissart (BE)
(86) Internationale Anmeldenummer: PCT/EP2005/010186
(87) Internationale Veröffentlichungsnummer: WO 2006/034805

(56) Entgegenhaltungen:
- EP-A- 0 011 244
- EP-A- 0 382 384
- WO-A1-91/01981

## Beschreibung

Die Erfindung betrifft ein Verfahren zur destillativen Reinigung von Tetrahydrofuran (THF) in Gegenwart von Methanol.

Die Herstellung von THF durch Dehydratisierung von 1,4-Butandiol an sauren Katalysatoren wird beispielsweise in DE-A 29 30 144 beschrieben und führt zu THF-Rohprodukten mit Wassergehalten von 18 bis 28 Gew.-% und Gehalten bis zu 5 Gew.-% von durch die Synthese bedingten Verunreinigungen wie 2,3-Dihydrofuran, 2- und 3-Methyltetrahydrofuran.

Für die Gewinnung von reinem THF aus derartigen Rohprodukten ist aus DE-A 37 26 805 ein Verfahren zu destillativen Reinigung von rohem wasserhaltigen THF bekannt, bei dem das THF durch drei Destillationskolonnen geleitet wird, wobei ein Seitenabzug der ersten Kolonne in die zweite geleitet wird, das Kopfprodukt der dritten Kolonne in die erste Kolonne zurückgeführt wird, am Kopf der ersten Kolonne Destillat entnommen wird und man das reine THF aus dem Seitenabzug der dritten Kolonne gewinnt.

Die Herstellung von THF durch Gasphasenhydrierung von Maleinsäureanhydrid (MSA) ist eine seit vielen Jahren bekannte Reaktion und ist beispielsweise in JP-B 2639463 und JP-B 2639464 beschrieben. Die erhaltenen wasserhaltigen Rohprodukte unterscheiden sich von den durch Butandiol-Dehydratisierung erhaltenen insbesondere durch ihren höheren Anteil an Komponenten, deren Siedepunkt sich vom Siedepunkt des THF nur wenig unterscheiden, sogenannten engsiedenden Komponenten. Unter den engsiedenden Komponenten des THF's sind insbesondere Methanol, Ethanol und Butyraldehyd, Butylmethylether, beziehungsweise deren Azeotrope mit THF und/oder Wasser zu nennen.

Durch die aus dem Stand der Technik bekannten Destillationsverfahren wird der Butylmethylether-Gehalt der wasserhaltigen THF-Rohprodukte der Gasphasenhydrierung von MSA oder MSA-Derivaten, oder der THF-haltige Rückführströme anderer Verfahren wie der Polytetrahydrofuran-Herstellung nicht ausreichend gesenkt, um die insbesondere bei der Weiterbearbeitung des THF's, beispielsweise zu Polytetrahydrofuran, an THF gestellten Reinheitsanforderungen zu erfüllen.

Es stellte sich daher die Aufgabe, ein verbessertes Verfahren zu finden, welches wirtschaftlich die Gewinnung von THF mit geringen Mengen an Butylmethylether-Gehalten aus THF ermöglicht. Dabei soll es unabhängig von der Herstellungsweise des THF's möglich sein, diese Reinheiten zu erreichen.

Gelöst wird diese Aufgabe durch ein Verfahren zur destillativen Reinigung von Tetrahydrofuran, in Gegenwart von Methanol, das bevorzugt vor Beginn der Destillation zugegeben wird. Gegenstand der Erfindung ist ein Verfahren zur destillativen Reinigung von Tetrahydrofuran in Gegenwart von Methanol, wobei die Destillation in einer Destillationskolonne durchgeführt wird.

Das erfindungsgemäße Verfahren ist auf THF, das durch unterschiedlichste Herstellungsverfahren und gegebenenfalls sich anschließende Reinigung des erhaltenen Rohprodukts beispielsweise nach dem aus DE-A 3726805 bekannten Verfahren, erzeugt wurde, anwendbar. Unter THF im Sinne dieser Anmeldung wird im allgemeinen THF verstanden, das einen Gehalt an THF > 99 Gew.-%, bis zu 1 Gew.-% Butylmethylether und kleiner 200 ppm weitere herstellungsbedingte sauerstofffunktionalisierte CH-Verbindungen aufweist. Bevorzugt wird das erfindungsgemäße Verfahren zur destillativen Trennung von Tetrahydrofuran eingesetzt, wie es bei der Tetrahydrofuran-Synthese durch katalytische Hydrierung von Maleinsäure (MSA) oder Maleinsäurederivaten (MSA-Derivaten) anfällt. Mit Maleinsäurederivaten sind Maleinsäureanhydrid, Fumarsäure, Maleinsäuremono- und diester, Furmarsäuremono- und diester, Bernsteinsäure, Bernsteinsäuremono- und diester und gamma-Bytrolacton, aber auch Maleinsäure selbst, gemeint. Die genannten Maleinsäurederivate können allein oder als Gemische, in Lösungsmitteln wie Wasser oder Alkoholen, in flüssiger Phase oder in der Gasphase katalytisch hydriert werden. Die Hydrierausträge können neben Tetrahydrofuran u.a. Butan-1,4-diol, gamma-Butyrolactoan, Alkohole wie Methanol, Ethanol, Propanol, Butanol, Wasser sowie unumgesetzte Maileinsäurederivate enthalten.
Es ermöglicht die Gewinnung eines reinen THF's, bei dem der Butylmethylether-Gehalt deutlich gesenkt wurde.

Die Destillation des THF's wird im allgemeinen in Gegenwart einer Menge von 0,1 bis 100 Gew.%, bezogen auf die Menge an THF, bevorzugt 0,1 bis 10 Gew.-%, besonders bevorzugt 0,1 bis 2 Gew.-%, von Methanol, das bevorzugt vor Beginn der Destillation zugegeben wird, durchgeführt.

Dabei kann das Methanol unverdünnt oder als Gemisch mit Wasser zugegeben werden. Vorzugsweise wird das Methanol als wässrige Lösung, wobei der Anteil des Methanols im Wasser von 0,1 bis 99,99 Gew.-%, bevorzugt von 99 bis 99,99 Gew.-% beträgt, zugegeben.

Die Destillation kann in einer von mehreren Stufen, beispielsweise in einer oder mehreren Destillationsstufen, beispielsweise in einer mehrstufigen Destillationskolonne im Gegenstrom, durchgeführt werden, wobei kontinuierlich oder diskontinuierlich gearbeitet werden kann. Bevorzugt wird die Destillation in zwei oder mehreren Destillationsstufen in Trennapparaturen, wie zum Beispiel Kolonnen mit mehreren Trennstufen, beispielsweise Rektifikationskolonnen, Füllkörperkolonnen, Glockenbödenkolonnen oder Packungskolonnen durchgeführt.

Besonders bevorzugt wird die Destillation in einer im Gegenstrom betriebenen Destillationskolonne, insbesondere bevorzugt in einer mehrstufigen Packungskolonne mit 1 bis 100 Trennstufen, bevorzugt 60 theoretischen Trennstufen kontinuierlich oder diskontinuirlich durchgeführt.

Als Packungsmaterialien kommen alle üblichen Kolonnenpackungen in Betracht. Bevorzugt werden jedoch Gewebepackungen verwendet.

Das Abdestillieren des Butylmethylether aus dem reinen Tetrahydrofuranprodukt erfolgt bei Drücken von 0 bis 100 bar, bevorzugt 0 bis 20 bar, besonders bevorzugt 0 bis 5 bar, bei Temperaturen von 10 bis 320°C.

Das erfindungsgemäße Verfahren wird nun in den nachfolgenden Beispielen näher erläutert.

### Beispiel 1

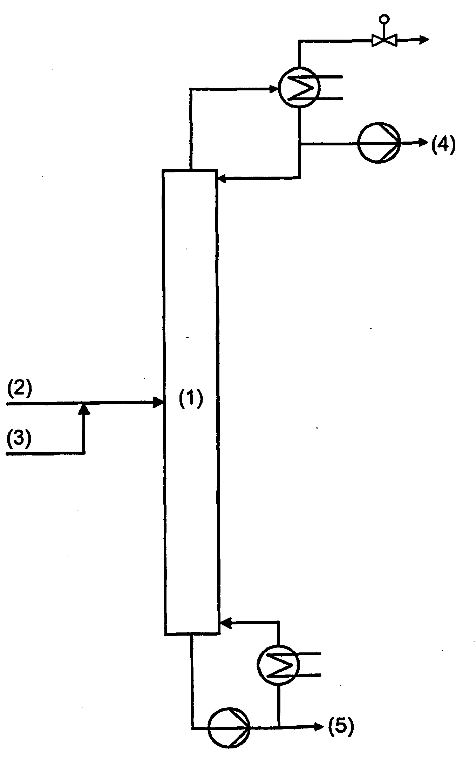

Es wurde die durch die Figur 1 dargestellte Destillationsapparatur verwendet. Die Kolonne (1) weist 60 theoretische Böden auf und wurde bei 2 bar absolut betrieben.

In die Kolonne wurde über die Zuleitung (2) ein THF-Gemisch bestehend aus 99,9 Gew.-% THF, 86 ppm Wasser, 24 ppm MeOH und 654 ppm Butylmethylether gefahren. Der Rest bis 100 Gew.-% wird von weiteren O-funktionalsierte CH-Verbindungen gebildet.

Gleichzeitig wurde in die Kolonnen-Zuleitung (2) über die Zuleitung (3) eines 0,65 Gew.-% Methanol, bezogen auf das THF-Gemisch aus Zuleitung (2), in Form eines Methanol/Wasser Gemisches, das 100 ppm Wasser enthielt, zugesetzt. Der Zulauf war im mittleren Drittel der Kolonne angebracht.

Die Kolonne (1) wurde mit Kopfabzug (4) und Sumpfabzug (5) betrieben. Das Rücklaufverhältnis bezogen auf den Zulaufstrom betrug 2,50 (Gew./Gew.). Aus dem Sumpfabzug (5) wurde aufgereinigtes THF abgeleitet.

Als Kopfabzug (4) wurde etwas mehr als 1,6 % der Zulaufmenge entnommen. Der Kopfabzug hatte eine THF-Konzentration von 59,1 Gew.-% THF, 39,2 Gew.-% MeOH, sowie 1,65 Gew.-% an Butylmethylether und geringe Mengen an Restwasser.

Das über den Sumpfabzug (5) gewonnene THF hatte 99,94 Gew.-% THF, 25 ppm MeOH, sowie nur noch 379 ppm Butylmethylether.

### Vergleichsbeispiel

Die in Beispiel 1 beschriebene Kolonne wurde unter gleichen Bedingungen betrieben. Allerdings wurde der Methanol/Wasser-Zulaufstrom (3) abgestellt, so dass kein Methanol zugeführt wurde.

Bei gleicher Feedmenge und einem Rücklaufverhältnis von 2,50 (Gew./Gew.) bezogen auf den Zulaufstrom, ergab sich folgendes Ergebnis:
Das über den Sumpfabzug (5) gewonnene THF hatte 99,92 Gew.-% THF und 659 ppm Butylmethylether. Der Gehalt an Butylmethylether ist gegenüber dem Ausgangsgemisch sogar leicht angestiegen.

Die nachfolgende Tabelle zeigt deutlich den Vorteil der Schleppmittelzugabe zur Abreicherung des Butylmethylether:

| | THF¹ [Gew.-%] | BME² [ppm] | MeOH³ [ppm] | BME-Abreicherung [%] |
|---|---|---|---|---|
| Zulaufgemisch (2) | 99,9 | 654 | 24 | |
| Beispiel 1 | 99,94 | 379 | 25 | - 42 |
| Vergleichsbeispiel | 99,92 | 659 | 0 | + 0,8 |

| | | | | |
|---|---|---|---|---|
| ¹ THF = Tetrahydrofuran ² BME = Butylmethylether ³ MeOH = Methanol | | | | |

## Patentansprüche

1. Verfahren zur destillativen Reinigung von Tetrahydrofuran in Gegenwart von Methanol, wobei die Destillation in einer Destillationskolonne durchgeführt wird

2. Verfahren nach einem der Anspruch 1, **dadurch gekennzeichnet, dass** das Lösungsmittel vor Beginn der Destillation zugegeben wird.

3. Verfahren nach einem der Ansprüche 1 oder 2, **dadurch gekennzeichnet, dass** in einer oder mehreren Destillationsstufen destilliert wird.

4. Verfahren nach einem der Ansprüche 1 bis 3, **dadurch gekennzeichnet, dass** in einer mehrstufigen Packungskolonne mit 1 bis 100 Trennstufen destilliert wird.

## Claims

1. A process for distillatively purifying tetrahydrofuran in the presence of methanol, wherein the distillation is conducted in a distillation column.

2. The process according to claim 1, wherein the solvent is added before the start of the distillation.

3. The process according to either of claims 1 and 2, wherein distillation is effected in one or more distillation stages.

4. The process according to any of claims 1 to 3, wherein distillation is effected in a multistage packed column having from 1 to 100 plates.

## Revendications

1. Procédé de purification de tétrahydrofuranne par distillation en présence de methanol, la distillation étant effectuée dans une colonne de distillation.

2. Procédé suivant la revendication 1, **caractérisé en ce que** le solvant est ajouté avant le début de la distillation.

3. Procédé suivant l'une quelconque des revendications 1 ou 2, **caractérisé en ce que** l'on distille en une ou plusieurs étapes de distillation.

4. Procédé suivant l'une quelconque des revendications 1 à 3, **caractérisé en ce que** l'on distille dans une colonne à remplissage à plusieurs étages, comportant de 1 à 100 étages de séparation.
